# DEMANDE DE BREVET EUROPEEN

(11) **EP 4 516 283 A1**
(43) Date de publication de la demande: **05.03.2025**
(21) Numéro de dépôt: 24198478.0
(22) Date de dépôt: 04.09.2024
(51) Int. Cl.: A61K 8/73, A61K 8/9789, A61Q 19/00, A61Q 19/08

(54) **PRINCIPE ACTIF COMPRENANT DES GALACTOMANNANES ISSUS DE MEDICAGO SATIVA ET SES UTILISATIONS COSMÉTIQUES**

(30) Priorité: 04.09.2023 FR 2309261
(71) Demandeur: Societe Industrielle Limousine d'Application Biologique, 19240 Saint Viance (FR)
(72) Inventeur: Paufique, Jean, 19130 Objat (FR)
(74) Mandataire: Aquinov

(57) **Abrégé**

La présente invention concerne un principe actif cosmétique comprenant des galactomannanes obtenus à partir des graines de *Medicago sativa* et ses utilisations cosmétiques. Ce principe actif présente un effet perfecteur de teint, il est mixte et multi-ethnique, destiné à tous types de peaux saines : caucasiennes, asiatiques, afro-américaines et hispano-américains, quel que soit leurs âges.

## Description

### Domaine technique

L'invention concerne un principe actif cosmétique comprenant des galactomannanes obtenus à partir des graines de *Medicago sativa* et ses utilisations cosmétiques. Ce principe actif présente avantageusement un effet perfecteur de teint mixte et multi-ethnique, c'est-à-dire destiné à tous types de peaux saines, qu'elles soient caucasiennes, asiatiques, afro-américaines, ou encore hispano-américains.

### Etat de l'art

Au fil du temps, la société n'a eu de cesse de faire évoluer les normes de la beauté idéale. Cependant, malgré les différences culturelles, l'industrie cosmétique est longtemps restée partisane de celles de l'Occident pour promouvoir ses produits. Même si les déclinaisons de teintes de maquillage ont vu le jour dès les années 70 aux Etats-Unis et ont ouvert les portes d'une beauté plus inclusive, ce n'est qu'avec la montée en puissance des réseaux sociaux et de ses influenceurs des quatre coins du monde que ce mouvement a entamé son réel essor. Portée par l'émergence de la diversité, de l'égalité et de l'inclusion, une re-priorisation des besoins individuels a progressivement dessiné un nouveau visage de la beauté où chaque individu ne se sent ni exclu, ni marginalisé. Ces principes vont de pair avec la demande accrue de soins ultra-personnalisés, motivée par toutes les catégories d'âges.

Cette approche s'étend désormais au-delà de la couleur de peau et tend à englober les caractéristiques spécifiques de chaque ethnie, chaque genre ou chaque étape de la vie. L'étude de ces spécificités structurelles et fonctionnelles de la peau a suscité un fort intérêt en dermatologie et a permis à l'industrie cosmétique d'exalter la « beauté plurielle ». Qu'elles soient masculines, féminines, jeunes, âgées ou originaires des quatre coins du monde, toutes les peaux ont leurs particularités et leurs problématiques

Néanmoins, hommes et femmes partagent des attentes communes en termes de soins hydratants et anti-âge, auxquelles s'ajoutent les soins perfecteur de teint. Un teint éclatant et homogène est donc une préoccupation universelle peu importe l'ethnie et le genre. Ainsi, en dépit de différences en termes de besoins et de préoccupations selon l'ethnie, l'âge et le sexe, trois axes majeurs de la beauté se dessinent pour répondre aux attentes universelles des consommateurs de soin de la peau : à savoir la qualité de la peau, le teint, et l'anti-âge.

Pour ces raisons, il existe un besoin pour un principe actif cosmétique permettant de répondre aux préoccupations et attentes de la beauté mixte et multi-ethnique.

Pour répondre à ce besoin, l'inventeur s'est particulièrement intéressé aux propriétés remarquables de l'alfalfa, déjà connu pour ses effets bénéfiques sur les peaux caucasiennes, pour développer un nouvel ingrédient dérivé du naturel répondant aux attentes de la beauté mixte et multi-ethnique.

Ainsi, l'inventeur a développé un nouveau principe actif cosmétique, à savoir un extrait de *Medicago sativa,* enrichi en galactomannanes permettant de conférer des bénéfices cosmétiques mixtes et multi-ethniques, en particulier un effet perfecteur de teint destiné à toutes peaux saines, qu'elles soient de type caucasienne, asiatique, afro-américaine, et hispano-américain.

### Résumé de l'invention

L'invention concerne ainsi, l'utilisation cosmétique d'un principe actif cosmétique comprenant au moins un hydrolysat comprenant des galactomannanes obtenu à partir des graines de *Medicago sativa,* pour son effet perfecteur de teint sur les peaux saines, préférentiellement les peaux saines caucasiennes et asiatiques et afro-américaines et hispano-américains.

Selon un objet préféré, l'invention porte sur l'utilisation cosmétique dudit principe actif cosmétique pour ses effets perfecteur de teint et hydratant mais également anti-âge. Ces effets cosmétiques combinés dans un seul et même principe actif cosmétique sont particulièrement d'intérêt pour répondre aux besoins de la beauté mixte et multi-ethnique.

Dans ce contexte, l'inventeur a évalué les bénéfices cosmétiques mixte et multi-ethniques de ce principe actif cosmétique, en particulier l'effet perfecteur de teint pour plusieurs types de peaux, à savoir les peaux caucasiennes et asiatiques et afro-américaines et hispano-américains. La performance du principe actif a ainsi été démontrée de la cellule au volontaire. Tout d'abord l'activité pro-collagène a été mesurée sur des fibroblastes issus de volontaires féminins et masculins, de différents âges et d'ethnies différentes. Le principe actif cosmétique selon l'invention a également démontré son pouvoir perfecteur de teint, c'est à dire une amélioration de l'éclat du teint ainsi que de l'homogénéisation du teint, notamment une diminution des désordres pigmentaires, sur tous les types de peau, répondant aux attentes des consommateurs.

Selon un autre aspect, l'invention vise également un nouveau principe actif cosmétique comprenant au moins un extrait obtenu à partir des graines de *Medicago sativa,* dans lequel l'extrait de *Medicago sativa* comprend, en poids du poids total de l'extrait, au moins 35% de sucres et au plus 25% de protéines.

Selon un objet préféré de l'invention, la fraction sucre de l'extrait comprend au moins 60% de galactomannanes en poids du poids total des sucres de l'extrait, l'extrait comprenant au moins 60% de galactomannanes sous forme d'oligo-galactomannanes ou poly-galactomannanes, de masses moléculaires comprises entre 360 Da et 1260 kDa, en poids du poids total des sucres de l'extrait, ce qui a pour effet d'améliorer les bénéfices cosmétiques pour chacun des consommateurs.

Plus préférentiellement, l'extrait selon l'invention comprend au moins 30% de galactomannanes de masses moléculaires comprises entre 3,6 kDa (DP20) et 1260 kDa (DP7000), en poids du poids total des sucres de l'extrait.

Le principe actif cosmétique comprenant l'extrait de *Medicago sativa* peut se présenter sous différentes formes : sous forme liquide ou sous forme solide ou sous forme de film, préférentiellement il est sous forme liquide. Celui-ci comprend avantageusement au moins un excipient choisi parmi un conservateur, un antioxydant, un stabilisant, un support d'atomisation, et/ou leur combinaison.

Selon un autre objet particulièrement préféré, l'extrait de *Medicago sativa* est un hydrolysat de *Medicago sativa,* plus préférentiellement un hydrolysat enzymatique.

Le principe actif selon l'invention peut être obtenu par différents procédés. Toutefois, selon un mode de réalisation particulièrement d'intérêt, le principe actif selon l'invention est obtenu par un procédé comprenant les étapes suivantes :
a. Solubilisation des graines de *Medicago sativa* dans l'eau à raison d'au moins 50 g/l,
b. Extraction par hydrolyse, préférentiellement hydrolyses enzymatiques,
c. Séparation des phases soluble et insoluble,
d. Traitement thermique,
e. Concentration de la phase soluble,
f. Purification par chromatographie ionique,
g. Éventuellement, filtration et filtration stérilisante.

Ainsi, l'invention se rapporte également au procédé d'extraction et de préparation du principe actif décrit précédemment.

D'autre part, l'invention vise également à une composition cosmétique comprenant au moins 0,1% d'un principe actif cosmétique selon l'un des quelconques objets précédents, en poids du poids total de la composition.

Enfin, l'invention se rapporte plus avantageusement à l'utilisation de ce principe actif cosmétique selon l'invention ou de la composition le comprenant, destiné à un traitement cosmétique pour les hommes et les femmes, en application topique sur la peau, préférentiellement sur la peau saine de type caucasienne, asiatique, afro-américaine, mais également hispano-américaine pour des bénéfices cosmétiques multiples, à savoir un effet perfecteur de teint, hydratant et anti-âge, peu importe le type de peau.

Aussi, l'inventeur a développé un principe actif d'origine naturel, mixte et multi-ethnique répondant aux attentes cosmétiques universelles en termes de beauté inclusive.

D'autres caractéristiques et avantages ressortiront de la description détaillée de l'invention, des exemples et des figures qui vont suivre.

### Brève description des Figures

La Figure 1 représente l'effet du principe actif selon l'invention formulé à 2,5% en émulsion sur le renouvellement cellulaire de volontaires jeunes caucasiens comparé à une formule placebo.
La Figure 2 représente l'effet du principe actif selon l'invention formulé à 2,5% en émulsion sur le renouvellement cellulaire de volontaires matures caucasiens comparé à une formule placebo.
La Figure 3 représente l'effet du principe actif selon l'invention formulé à 2,5% en émulsion sur la qualité des fibres de la matrice dermique de volontaires caucasiens matures après 42 jours de traitement biquotidien.
La Figure 4 représente l'effet du principe actif selon l'invention formulé à 2,5% en émulsion sur le paramètre rayonnement de l'éclat du teint de panels jeunes et matures, 15 minutes après une application unique ainsi qu'après 7, 21 ou 42 jours de traitement.

### Description détaillée de l'invention

### Définition

Par « beauté inclusive », « beauté universelle » ou « beauté mixte et multi-ethnique » au sens de la présente invention, on entend une amélioration de la qualité de la peau, de l'éclat du teint, de l'homogénéité du teint, et de l'hydratation de la peau, peu importe que la peau saine soit de type caucasienne, asiatique, afro-américaine, ou hispano-américain, qu'elle concerne les hommes ou les femmes et quel que soit leur âge.

Par « extrait de *Medicago sativa* » au sens de la présente invention, on entend au moins une molécule ou un mélange d'au moins deux molécules obtenu(es) à partir d'une matière première, à savoir l'alfalfa, c'est à dire l'espèce *Medicago sativa,* quel que soit le procédé d'extraction de ladite ou desdites molécule(s). Il peut s'agir par exemple d'un extrait obtenu par extraction aqueuse et/ou hydroalcoolique et/ou hydroglycolique et/ou obtenu après au moins une étape d'hydrolyse, par exemple enzymatique ou acide.

Par « hydrolysat de *Medicago sativa* » au sens de l'invention, on entend un extrait issu de l'espèce végétale *Medicago sativa,* obtenu par un procédé comprenant au moins une étape d'hydrolyse de *Medicago sativa.* Le terme hydrolysat de *Medicago sativa* exclut les molécules produites uniquement par fermentation de *Medicago sativa* par un micro-organisme ou une infusion/décoction/macération de *Medicago sativa.* Ainsi, au sens de l'invention un tel extrait est un ingrédient dérivé du naturel obtenu par des procédés chimiques et/ou biologiques spécifiques et définis visant à le modifier chimiquement, modifications qui sont intentionnelles, et qui permettent d'obtenir des structures de molécules qui ne sont donc plus identiques aux molécules présentes dans la nature.

Par « principe actif » au sens de l'invention, on entend un extrait comprenant au moins une molécule, préférentiellement un ensemble de molécules présentant un effet cosmétique notamment lorsqu'il est appliqué topiquement, en particulier l'effet cosmétique est un effet perfecteur de teint.

Par « perfecteur de teint » au sens de l'invention, on entend une amélioration de l'éclat du teint et une diminution des marques d'hyperpigmentation post-inflammation de la peau.

### Principe actif selon l'invention

La présente invention a donc pour objet un principe actif cosmétique comprenant au moins un extrait obtenu à partir des graines de *Medicago sativa,* dans lequel l'extrait de *Medicago sativa* comprend, en poids du poids total de l'extrait, au moins 35% de sucres et au plus 25% de protéines.

*Medicago sativa* également connu sous le terme alfalfa ou luzerne est une plante fourragère originaire d'Asie dont la culture s'est développée à travers le monde, notamment pour ses qualités nutritionnelles et ses propriétés médicinales. Elle se pare de grappes de fleurs arborant différentes nuances de violet qui se développent ensuite en des fruits prenant la forme de gousses spiralées, ceux-ci comprennent les graines. En outre, les racines abritent des microorganismes établissant une symbiose fixatrice d'azote, aidant à la fois au développement de la plante et à l'enrichissement du sol. La culture de l'alfalfa revêt donc une grande importance écologique pour l'environnement et la biodiversité, en sus de ses propriétés nutritionnelles et médicinales.

L'accès (selon la définition du Protocole de Nagoya) à la matière première végétale *(Medicago sativa)* a été effectué conformément à la règlementation nationale APA (Accès et Partage des Avantages) du pays d'origine, la France.

Les graines d'alfalfa sont naturellement riches en polysaccharides de type polymères de mannoses et de galactoses que l'inventeur a remarquablement exploité de façon à obtenir un extrait enrichi en galactomannanes d'intérêt.

Le principe actif selon l'invention comprenant l'extrait de *Medicago sativa* est préférentiellement composé majoritairement de galactomannanes, plus préférentiellement l'extrait comprend au moins 60% de galactomannanes, en poids du poids total des sucres totaux compris dans l'extrait. Cette composition a été déterminée par chromatographie ionique.

L'inventeur a caractérisé les sucres présents dans l'extrait selon l'invention par chromatographie (LC-CAD). La teneur en sucres totaux, quant à elle, peut être déterminée selon la méthode de DUBOIS (Dubois M. et al., Analytical chemistry, 28, 3, 350-356, 1956).

Selon un objet préféré, l'extrait selon l'invention comprend au moins 60% de sucres de masses moléculaires comprises entre 360 Da et 1260 kDa (DP2 à DP7000), en poids du poids total des sucres de l'extrait.

Selon un autre objet préféré de l'invention, l'extrait selon l'invention comprend au moins 30% de sucres de masses moléculaires comprises entre 3,6 kDa (DP20) et 1260 kDa (DP7000), en poids du poids total des sucres de l'extrait.

Très préférentiellement, l'extrait selon l'invention comprend au moins 60% de sucres de masses moléculaires comprises entre 360 Da et 1260 kDa (DP2 et DP7000), en poids du poids total des sucres de l'extrait, ainsi qu'au moins 30% de sucres de masses moléculaires comprises entre 3,6 kDa (DP20) et 1260 kDa (DP7000), en poids du poids total des sucres de l'extrait.

Enfin, selon un autre objet, l'extrait comprend au plus 25% de sucres de masses moléculaires inférieures ou égales à 180 Da (DP1), en poids du poids total des sucres de l'extrait, préférentiellement au plus 20% de sucres de masses moléculaires inférieures ou égales à 180 Da.

La composition des sucres constituant l'extrait selon l'invention a également été déterminée, celle-ci peut notamment être déterminée par chromatographie ionique. L'extrait comprend avantageusement du galactose, mannose, glucose et fructose. Très avantageusement, il comprend majoritairement du galactose et du mannose.

L'extrait selon l'invention est également riche en galactomannanes. Pour améliorer et optimiser cet enrichissement, l'extrait est très préférentiellement obtenu à l'aide d'une hydrolyse, encore plus préférentiellement au moins une hydrolyse enzymatique. Ainsi, l'extrait de *Medicago sativa* selon l'invention est préférentiellement un hydrolysat, plus préférentiellement un hydrolysat enzymatique.

L'extrait selon l'invention peut être obtenu par tout procédé d'extraction. Toutefois, de façon particulièrement préférée, l'extrait est susceptible d'être obtenu par un procédé comprenant au moins les étapes suivantes :
a. Solubilisation des graines de *Medicago sativa* dans l'eau à raison d'au moins 50 g/l,
b. Extraction par hydrolyse, préférentiellement hydrolyses enzymatiques,
c. Séparation des phases soluble et insoluble,
d. Traitement thermique,
e. Concentration de la phase soluble,
f. Purification par chromatographie ionique,
g. Éventuellement, filtration et filtration stérilisante.

Le principe actif cosmétique selon l'invention peut également avantageusement comprendre au moins un excipient choisi parmi un conservateur, un antioxydant, un stabilisant, un support d'atomisation, et/ou leur combinaison.

L'extrait selon l'invention présent dans le principe actif cosmétique peut alors se présenter sous forme liquide ou sous forme solide ou sous forme de film.

Lorsqu'il se présente sous forme liquide, le principe actif selon l'invention est exclusivement constitué par l'extrait de *Medicago sativa* accompagné de stabilisant et/ou système de conservation.

L'extrait sous forme liquide se présente préférentiellement sous forme d'une solution aqueuse liquide limpide, avec une faible odeur et une couleur jaune claire à jaune.

Lorsqu'il se présente sous forme liquide, la teneur en matières sèches de l'extrait peut être déterminée par la pesée des résidus issus du séchage de l'extrait selon l'invention à 105°C dans une étuve jusqu'à l'obtention d'un poids constant. Préférentiellement, l'extrait selon l'invention sous forme liquide a une teneur en matières sèches de 20 g/L à 70 g/L, préférentiellement de 20 à 55 g/L, encore plus préférentiellement de 22 g/L à 32 g/L.

Lorsqu'il se présente sous forme solide, le principe actif selon l'invention est préférentiellement constitué par l'extrait de *Medicago sativa* tel que précédemment décrit et par un support choisi parmi la maltodextrine, la gomme arabique ou la lécithine de soja. Selon un mode de réalisation, particulièrement adapté, l'extrait représente au moins 10% en poids du principe actif et le support au plus 90% en poids du principe actif.

Dans le cas d'une forme solide dans laquelle le principe actif est associé à un support, les teneurs en protéines, en sucres et en galactomannanes par rapport à la matière sèche dans le principe actif peuvent être modifiées puisque les supports (maltodextrine, gomme arabique) sont exclusivement des saccharides.

Le principe actif selon l'invention peut aussi être présenté sous forme d'un film tel que décrit dans le brevet FR3079145. Dans ce cas, l'extrait de *Medicago sativa* représente préférentiellement au moins 0,1% en poids du film.

Lorsqu'il se présente sous forme de film, le principe actif comprend :
- au moins l'extrait de *Medicago sativa* selon l'invention.
- au moins une charge minérale, et
- au moins un polymère d'origine naturelle, et
- au moins un plastifiant, et
- au moins un tensio-actif.

Le polymère d'origine naturelle peut être choisi parmi : Pectine, Gomme tamarin, Alginate, Pullulane, Psyllium, Xanthane, Guar, Tara, Caroube, Agar, Gomme arabique, Gellane, Dextran, Carraghénane, Cellulose, Konjac et Chitosan.

Le plastifiant peut être choisi parmi : Glycérol, Sorbitol, Saccharose, Erythritol, Urée, Propylène glycol et le Butylène glycol.

La charge minérale peut être choisie parmi : carbonate de calcium, argile verte, kaolin, perlite, talc, silicate de magnésium, mica, sericite diatomée, silice, sulfate de calcium, chlorure de calcium, chlorure de potassium, oxyde de fer et oxyde de zinc.

Le principe actif peut comprendre, en outre, un pigment pour colorer le film.

### Composition selon l'invention

Le principe actif selon l'invention a pour but d'être intégré dans une composition, notamment une composition comprenant au moins 0,1% en poids dudit principe actif et un milieu physiologiquement acceptable, préférentiellement un milieu cosmétiquement acceptable.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux, poudres, ou fond de teint ou sous forme de film. Elles peuvent être plus ou moins fluides et avoir l'aspect de crèmes, émulsions, gels, masques ou tous autres aspects connus de l'Homme du métier.

Préférentiellement, il peut s'agir de compositions comprenant entre 0,1 et 20% du principe actif selon l'invention, notamment sous forme liquide, plus préférentiellement entre 0,5 et 10%.

Ces compositions comprennent, outre le principe actif, un milieu physiologiquement acceptable tel qu'un milieu cosmétiquement acceptable, c'est-à-dire qui ne provoque pas de sensations d'inconfort pour l'utilisateur telles que des rougeurs, tiraillements ou picotements.

Les compositions selon l'invention peuvent également contenir comme ingrédient au moins un composé choisi parmi :
- les huiles, qui peuvent être choisies notamment parmi les huiles de silicone, linéaires ou cycliques, volatiles ou non volatiles,
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels ingrédients sont cités notamment dans le Dictionnaire CTFA (International Cosmetic ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

### Procédé de préparation de l'extrait selon l'invention

L'extrait constituant ou contenu dans le principe actif selon l'invention peut être obtenu par tout moyen.

Toutefois, selon un mode de réalisation préféré, le procédé d'extraction comprend au moins une étape d'extraction à partir de *Medicago sativa,* tel qu'au moins une hydrolyse, préférentiellement au moins une hydrolyse enzymatique, plus préférentiellement au moins une hydrolyse enzymatique à l'aide d'une carbohydrase.

Selon un mode de réalisation particulièrement adapté, le principe actif selon l'invention est obtenu par la mise en oeuvre des étapes suivantes :
a. Solubilisation des graines de *Medicago sativa* dans l'eau à raison d'au moins 50 g/l,
b. Extraction par hydrolyse, préférentiellement hydrolyses enzymatiques,
c. Séparation des phases soluble et insoluble,
d. Traitement thermique,
e. Concentration de la phase soluble contenant les galactomannanes,
f. Purification par chromatographie ionique afin d'enrichir en galactomannanes,
g. Éventuellement, filtration et filtration stérilisante.

L'étape de traitement thermique est avantageusement réalisée pour inactiver la ou les enzyme(s) présente(s). Cette inactivation est alors réalisée selon les préconisations du fournisseur de la ou des enzyme(s) utilisée(s).

La séparation de la phase soluble et insoluble est réalisée par tout moyen connu de l'homme du métier, par exemple par centrifugation, filtration ou décantation. La séparation des phases soluble et insoluble est réalisée pour récupérer la phase soluble contenant les galactomannanes.

L'extrait obtenu à ce stade peut éventuellement être encore concentré. Préférentiellement l'extrait est purifié, préférentiellement par des étapes d'ultrafiltration successives à travers des membranes de porosité différentes, en conservant les galactomannanes à chaque étape et/ou par une méthode de type chromatographique. Dans le contexte de l'invention, l'extrait obtenu après l'étape de concentration de la phase soluble, subit au moins une étape de purification afin d'enrichir la teneur en galactomannanes, préférentiellement la purification est mise en oeuvre au moyen d'une chromatographie ionique.

L'extrait obtenu après hydrolyse et concentration est un extrait de *Medicago sativa,* et constitue une première forme du principe actif selon l'invention, se présentant alors sous forme liquide.

Une autre forme du principe actif selon l'invention est obtenue après hydrolyse, concentration et purification et se présente également sous forme liquide.

L'extrait obtenu peut ensuite être séché et associé ou non à un support, pour se présenter sous forme solide. Cette phase peut être réalisée par la mise en oeuvre des étapes suivantes :
- un support d'atomisation, de préférence la maltodextrine, est ajouté dans l'extrait de *Medicago sativa,* d'au plus 90% (en masse/volume) ;
- cette solution est ensuite concentrée sous vide ;
- un traitement antimicrobien peut être réalisé ;
- l'atomisation permet d'obtenir une poudre.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

### Utilisations cosmétiques

Selon un aspect, l'invention vise l'utilisation cosmétique d'un principe actif cosmétique comprenant au moins un hydrolysat comprenant des galactomannanes obtenu à partir des graines de *Medicago sativa,* pour son effet perfecteur de teint sur les peaux saines multi-ethniques.

Préférentiellement, les bénéfices cosmétiques de ce principe actif cosmétique comprenant au moins un hydrolysat enrichi en galactomannanes obtenu à partir des graines de *Medicago sativa,* sont dits mixtes et multi-ethniques. Aussi, l'invention vise préférentiellement l'utilisation cosmétique de ce principe actif, pour les peaux saines caucasiennes et asiatiques et afro-américaines et hispano-américains. En effet, l'inventeur a observé dans le cadre de ses travaux, qu'un tel principe actif permettait de répondre aux besoins de la beauté mixte et multi-ethnique.

Plus préférentiellement, l'invention vise l'utilisation cosmétique de ce principe actif pour ses effets perfecteur de teint et hydratant et anti-âge.

Pour y parvenir, ce principe actif comprenant au moins un hydrolysat enrichi en galactomannanes obtenu à partir des graines de *Medicago sativa,* est avantageusement un hydrolysat comprenant au moins 60% de galactomannanes en poids du poids total des sucres de l'hydrolysat.

Selon une variante, le principe actif selon l'un des quelconques objets précédemment décrits, ou de la composition le comprenant est destiné à être utilisé sur tous type de peaux saines, sans distinction d'ethnie de genre ou d'âge. Celui-ci présente alors un effet perfecteur de teint, préférentiellement des effets perfecteur de teint, hydratant et anti-âge sur tous types de peaux saines. Ainsi, l'invention se rapporte également à l'utilisation cosmétique du principe actif selon l'invention comprenant au moins un extrait obtenu à partir des graines de *Medicago sativa,* ledit extrait comprenant au moins 35% de sucres et au plus 25% de protéines, pour son effet perfecteur de teint sur tous types de peaux saines, préférentiellement ses effets cumulés perfecteur de teint, hydratant et anti-âge.

Le principe actif selon l'invention permet ainsi de répondre aux besoins de la beauté dite mixte et multi-ethnique. En effet, le principe actif selon l'invention ou la composition selon l'invention agit préférentiellement sur tous types de peaux, en particulier les peaux caucasiennes et asiatiques et afro-américaines et hispano-américains.

Ainsi, selon un objet particulièrement d'intérêt, l'invention se rapporte à l'utilisation cosmétique du principe actif selon l'un des quelconques modes de réalisation précédemment décrit ou d'une composition le comprenant, destiné à un traitement cosmétique en application topique sur la peau, pour ses effets perfecteur de teint, ou ses effets cumulés perfecteur de teint, hydratant et anti-âge sur des peaux saines de type caucasienne et asiatique et afro-américaine et hispano-américaine.

L'invention est à présent illustrée par des exemples non limitatifs de compositions selon l'invention et par des résultats.

### Exemples

### Exemple 1 : Principe actif selon l'invention

Le principe actif de l'exemple 1 est obtenu à partir de la luzerne de l'espèce *Medicago sativa.* Le principe actif de l'exemple 1 est obtenu par le procédé suivant :
a. Solubilisation des graines de *Medicago sativa* dans l'eau à raison d'au moins 50 g/l,
b. Extraction par hydrolyses enzymatiques,
c. Séparation des phases soluble et insoluble,
d. Traitement thermique,
e. Concentration de la phase soluble,
f. Purification par chromatographie ionique,
g. Éventuellement, filtration et filtration stérilisante.

Le principe actif selon l'exemple 1 est constitué d'un extrait de *Medicago sativa* possédant notamment les caractéristiques suivantes :
- Teneur en Matières Sèches = 26,9 g/L, dont :
- Teneur en protéines = 5,2 g/l soit 19%, en poids par rapport à la matière sèche,
- Teneur en minéraux = 9% en poids par rapport à la matière sèche,
- Teneur en sucres totaux = 20,1 g/L, soit 72% en poids par rapport à la matière sèche,
- 49% des sucres ont une masse moléculaire comprise entre 360 et 3 600 Da et 34% des sucres ont une masse moléculaire comprise entre 3 600 Da et 1260 kDa,
   - les sucres sont constitués de 45,9% galactose, 36,6% de mannose, 14,4% glucose et 3,1% de fructose.

La teneur en minéraux a été déterminée par la pesée des résidus issus de l'incinération à 550°C dans un four à moufle électrique permettant d'obtenir la teneur en cendres minérales. La teneur en protéines a été déterminée selon la méthode de LOWRY (Lowry et al., Protein measurement with the folin reagent, J. Biol. Chem., 193, 265, 1951).

### Evaluation de l'efficacité du principe actif selon l'invention

### Essai 1 - Evaluation in vitro de l'efficacité du principe actif selon l'invention sur les fonctionnalités épidermiques.

L'objectif de cette étude est d'évaluer l'efficacité du principe actif selon l'invention sur les fonctionnalités épidermiques. Le maintien de la barrière épidermique permet de protéger la peau. Or, plusieurs composants protéiques sont indispensables à l'établissement et au maintien de cette fonction barrière, à savoir :
- le Ki-67, marqueur-clé de la prolifération des cellules de la couche basale de l'épiderme ;
- la loricrine, protéine jouant un rôle phare dans la différenciation terminale ;
- la claudine-1, constituant majeur des jonctions serrées responsables de la cohésion cellulaire.

L'homéostasie hydrique est également un élément indispensable à l'équilibre physiologique de la peau. Son maintien est garanti par un ensemble de régulations fines s'appuyant notamment sur des marqueurs biologiques spécifiques tels que l'aquaporine 3.

Enfin, la jonction dermo-épidermique assure plusieurs fonctions fondamentales au sein de la peau. Elle a notamment un rôle de support mécanique pour l'adhésion de l'épiderme au derme et de barrière sélective permettant le contrôle des échanges moléculaires et cellulaires entre ces deux compartiments. Cette jonction dermo-épidermique est constituée de plusieurs protéines impliquées dans le maintien de son intégrité comme :
- l'intégrine b4, molécule transmembranaire au niveau des hémidesmosomes et récepteur de la laminine ;
- la laminine 332 présente au niveau des filaments d'ancrage ;
- le collagène VII situé au niveau des fibrilles d'ancrage.

Par conséquent, cette étude vise à évaluer ces différents marqueurs. Elle a été réalisée par immunohistofluorescence et par coloration sur des peaux reconstruites jeunes et âgées pour la coloration des lipides, en présence d'une sonde fluorescente. Le marquage par immunohistofluorescence a été réalisé avec des anticorps anti-Ki-67, anti-loricrine, anti-claudine-1, anti-aquaporine 3, anti-intégrine β4, anti-laminine 332 et anti-collagène VII, permettant de mesurer la synthèse de chaque protéine.

Les résultats sont présentés dans les tableaux 1 et 2, décrivant respectivement les résultats sur la barrière physique et hydrique, ainsi que sur l'ancrage de l'épiderme au derme.

**[Tableau 1]**

| | **Peaux reconstruites jeunes** | | **Peaux reconstruites âgées** | |
|---|---|---|---|---|
| | **Témoin** | **INVENTION 0,5%** | **Témoin** | **INVENTION 0,5%** |
| **Ki-67** | | | | |
| Cellules Ki-67 positives (%) | 42 | 56 | 27 | 44 |
| Capacité à augmenter la synthèse (%) | | **-33** | | |
| Capacité à restaurer la synthèse (%) | | | | **+113** |

| **Loricrine** | | | | |
|---|---|---|---|---|
| Synthèse (×10⁴ UA) | 490 | 589 | 255 | 368 |
| Capacité à augmenter la synthèse (%) | | **+20** | | |
| Capacité à restaurer la synthèse (%) | | | | **+48** |

| **Claudine-1** | | | | |
|---|---|---|---|---|
| Synthèse (×10⁴ UA) | 345 | 424 | 205 | 338 |
| Capacité à augmenter la synthèse (%) | | **+23** | | |
| Capacité à restaurer la synthèse (%) | | | | **+95** |

| **Lipides neutres** | | | | |
|---|---|---|---|---|
| Synthèse (×10⁵ UA) | 240 | 413 | 61 | 101 |
| Capacité à augmenter la synthèse (%) | | +72 | | |
| Capacité à restaurer la synthèse (%) | | | | **+22** |

| **Aquaporine 3** | | | | |
|---|---|---|---|---|
| Synthèse (×10⁵ UA) | 201 | 255 | 109 | 180 |
| Capacité à augmenter la synthèse (%) | | +27 | | |
| Capacité à restaurer la synthèse (%) | | | | **+77** |

**[Tableau 2]**

| | **Peaux reconstruites jeunes** | | **Peaux reconstruites âgées** | |
|---|---|---|---|---|
| | **Témoin** | **INVENTION 0,5%** | **Témoin** | **INVENTION 0,5%** |
| **Intégrine b4** | | | | |
| Synthèse (×10⁴ UA) | 456 | 585 | 309 | 380 |
| Capacité à augmenter la synthèse (%) | | **+28** | | |
| Capacité à restaurer la synthèse (%) | | | | **+48** |

| Laminine 332 | | | | |
|---|---|---|---|---|
| Synthèse (×10⁵ UA) | 539 | 643 | 481 | 592 |
| Capacité à augmenter la synthèse (%) | | **+19** | | |
| Capacité à restaurer la synthèse (%) | | | | **+191** |

| **Collagène VII** | | | | |
|---|---|---|---|---|
| Synthèse (×10⁴ UA) | 110 | 134 | 72 | 88 |
| Capacité à augmenter la synthèse (%) | | **+22** | | |
| Capacité à restaurer la synthèse (%) | | | | **+42** |

Testé à 0,5% sur des peaux reconstruites jeunes et âgées, le principe actif selon l'invention augmente significativement la synthèse des protéines de la barrière physique et hydrique, ainsi que celles impliquées dans l'ancrage de l'épiderme au derme. Le principe actif selon l'invention permet ainsi de maintenir une barrière épidermique fonctionnelle.

### Essai 2 - Evaluation in vitro de l'efficacité du principe actif selon l'invention sur l'activité pro-collagène mixte et multi-éthnique.

L'objectif de cette étude est d'évaluer la capacité du principe actif selon l'invention à renforcer et préserver la matrice dermique.

L'étude a été réalisée par dosage ELISA sur des fibroblastes humains, soumis ou non à une exposition aux UVA. Les fibroblastes sont issus de femmes ou d'hommes, jeunes ou matures, et d'ethnies différentes (caucasien, asiatique, afro-américain).

Le protocole est le suivant. Les fibroblastes humains sont ensemencés et incubés à 37°C, puis ils sont traités avec le principe actif selon l'invention à 0,25%, 0,50% et 1,00% (V/V). Les cellules sont ensuite incubées à 37°C et les surnageants sont récupérés et stockés à -80°C en attente du dosage ELISA. Pour l'étude du taux de MMP-1, les fibroblastes sont irradiés avec une lampe UV.

Les résultats sont présentés dans les tableaux 3 et 4 ci-après.

**[Tableau 3]**

| | **Synthèse de collagène I (ng/mg protéines)** | | **Efficacité (%)** |
|---|---|---|---|
| | Témoin | INVENTION 1,00% | **INVENTION 1,00% / Témoin** |
| **Caucasien** | | | |
| Femmes jeunes | 354 | 1509 | **+326** |
| Hommes jeunes | 299 | 1127 | **+277** |
| Femmes matures | 294 | 1231 | **+319** |
| Hommes matures | 357 | 1786 | **+400** |

| **Asiatique** | | | |
|---|---|---|---|
| Femmes matures | 261 | 788 | **+202** |
| Homme matures | 258 | 844 | **+227** |

| **Afro-américain** | | | |
|---|---|---|---|
| Femme jeunes | 294 | 711 | **+142** |

**[Tableau 4]**

| | **Synthèse de MMP-1 (ng/mg protéines)** | | | **Capacité à limiter la synthèse de MMP-1 (%)** |
|---|---|---|---|---|
| | **Fibroblastes non irradiés** | **Fibroblastes irradiés** | | |
| | Témoin | Témoin | INVENTION 1,0% | **INVENTION 1,0% / Témoin** |
| **Caucasien** | | | | |
| Femmes jeunes | 10 | 99 | 63 | **+40** |
| Hommes jeunes | 5 | 106 | 77 | **+29** |
| Femmes matures | 2 | 62 | 30 | **+53** |
| Hommes matures | 13 | 69 | 45 | **+43** |

| **Asiatique** | | | | |
|---|---|---|---|---|
| Femmes matures | 28 | 76 | 61 | **+31** |
| Hommes matures | 8 | 103 | 51 | **+55** |

| **Afro-américain** | | | | |
|---|---|---|---|---|
| Femmes jeunes | 1 | 11 | 6 | **+50** |

Testé à 1%, sur fibroblastes humains mixtes, de différents âges et différentes ethnies, le principe actif selon l'invention stimule significativement la synthèse du collagène I et limite significativement la synthèse de MMP-1 indifféremment du sexe, de l'âge et de l'ethnie. Ainsi, le principe actif selon l'invention préserve et renforce la matrice dermique quel que soit le type de peau.

### Essai 3 - Evaluation in vivo de l'efficacité du principe actif selon l'invention.

Les activités biologiques et les bénéfices cosmétiques du principe actif selon l'invention à 2,5% en émulsion ont été évalués *in vivo,* chez des panels mixtes, composés de sujets jeunes et matures issus d'ethnies différentes.

La composition des panels des études sur le visage est détaillée dans le tableau 5 ci-après.

**[Tableau 5]**

| **Panels jeunes** | **Produits testés et modalités d'application** | **Nombre de sujets / âge moyen** |
|---|---|---|
| Caucasien | - formule placebo | - 20 sujets sains : (2 hommes et 18 femmes) |
| | -formule contenant le principe actif selon l'invention à 2,5% | |
| | | - âge moyen 36 ans |
| | 21 jours d'application biquotidienne en hémi-visage | |
| Hispano-américain | - formule placebo | Groupe placebo : |
| | -formule contenant le principe actif selon l'invention à 2,5% | - 29 sujets sains (9 hommes et 20 femmes) |
| | 21 jours d'application biquotidienne en visage entier | - âge moyen 23 ans |
| | | Groupe INVENTION : |
| | | - 25 sujets sains (6 hommes et 19 femmes) |
| | | - âge moyen 23 ans |
| | | |
| Afro-américain | -formule contenant le principe actif selon l'invention à 2,5% | Groupe INVENTION : |
| | | - 30 sujets sains (6 hommes et 24 femmes) |
| | 21 jours d'application biquotidienne en visage entier | - âge moyen 34 ans |

| **Panels matures** | **Produits testés et modalités d'application** | **Nombre de sujets / âge moyen** |
|---|---|---|
| Caucasien | - formule placebo | Groupe placebo : |
| | -formule contenant le principe actif selon l'invention à 2,5% en émulsion | - 19 sujets sains (1 homme et 18 femmes) |
| | | - âge moyen 64 ans |
| | 42 jours d'application biquotidienne en visage entier | Groupe INVENTION : |
| | | - 19 sujets sains (19 femmes) |
| | | - âge moyen 64 ans |
| Asiatique | - formule placebo | - 34 sujets sains (34 femmes) |
| | | - âge moyen 55 ans |
| | -formule contenant le principe actif selon l'invention à 2,5% | |
| | 42 jours d'application biquotidienne en hémi-visage | |

### Essai 3.1 - Evaluation de la qualité de la fonction barrière sur le panel jeune caucasien

Pour évaluer la qualité de la fonction barrière, les pertes insensibles en eau ont été mesurées à l'aide d'un Tewamètre. Les résultats sont présentés dans le tableau 6 ci-après.

**[Tableau 6]**

| | Variation / J0 (%) | |
|---|---|---|
| | J7 | J21 |
| Placebo | 4,2 | 6,2 |
| Principe actif 2,5% | -8,5 | -12,6 |
| Variation / Placebo (%) | -12,6 | -18,8 |

Dès 7 jours d'application biquotidienne, le principe actif selon l'invention formulé à 2,5% améliore significativement la qualité de la barrière cutanée des volontaires caucasiens jeunes à travers la réduction des pertes en eau. Cet effet s'intensifie après 21 jours de traitement. Le principe actif selon l'invention favorise ainsi le renforcement de la fonction barrière.

### Essai 3.2 - Evaluation de la capacité à réduire l'oxydation des protéines sur le panel jeune caucasien

Les protéines oxydées ont été étudiées par marquage fluorescent sur strippings. Après 7 jours de traitement biquotidien du principe actif selon l'invention à 2,5%, la quantité de protéines oxydées présentes à la surface de la peau de sujets caucasiens jeunes a diminué de 11,1%. Cet effet se maintient après 21 jours de traitement avec une réduction du taux de protéines oxydées de 10,5%.

En réduisant la formation de protéines oxydées, le principe actif selon l'invention améliore la transmission de la lumière à travers le *stratum corneum* et participe ainsi à l'amélioration de l'éclat de la peau.

### Essai 3.3 - Evaluation du renouvellement cellulaire sur le panel jeune et mature caucasien

L'évaluation du renouvellement cellulaire a été réalisée sur la face interne des avant-bras d'un panel composé de 19 sujets d'âge moyen 34 ans (3 hommes et 16 femmes) et 17 sujets d'âge moyen 67 ans (1 homme et 16 femmes). Cette étude a été réalisée à partir de photographies prises à intervalles réguliers pendant 14 jours, à l'aide d'un dermoscope C-Cube. Le principe actif selon l'invention à 2,5% en émulsion et la formule placebo ont été appliquée sur la face interne des avant-bras sur une zone préalablement définie et colorée à l'aide d'une formule contenant de la dihydroxyacétone (DHA) :
- 14 jours précédant la coloration (phase de pré-traitement).
- 14 jours suivant la coloration (phase de traitement).

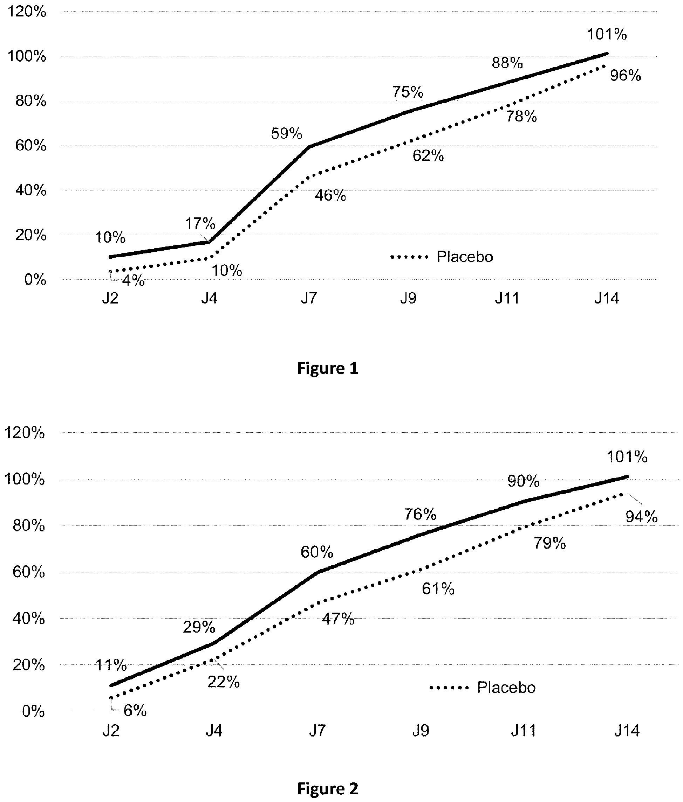

Les résultats sont présentés en figure 1 et en figure 2.

Le principe actif selon l'invention à 2,5% stimule le renouvellement de l'épiderme aussi bien chez des volontaires caucasiens jeunes que matures : la DHA est éliminée plus rapidement après traitement avec le principe actif selon l'invention en comparaison à la formule placebo. Celui-ci favorise donc le renouvellement épidermique des volontaires caucasiens jeunes et matures en augmentant significativement la capacité de leur peau à éliminer la DHA dès 2 jours après son application.

### Essai 3.4 - Evaluation de la qualité de la matrice dermique sur le panel caucasien mature

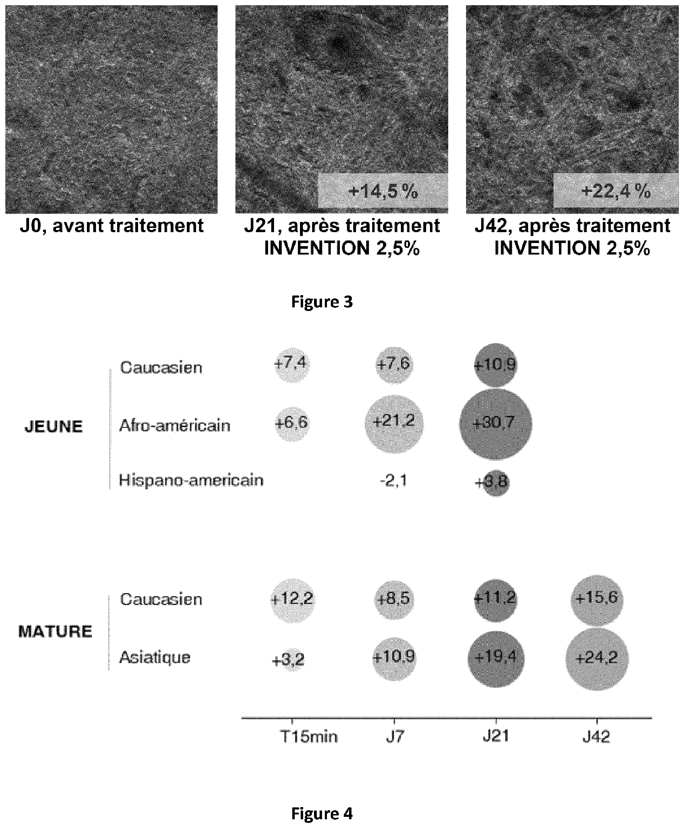

L'étude de la qualité des fibres a été réalisée par LC-OCT (résultats présentés en Figure 3) et l'étude de la densité du derme a été réalisée par échographie haute fréquence.

Les résultats de l'échographie haute fréquence sont présentés dans le tableau 7 ci-après.

**[Tableau 7]**

| | Variation / J0 (%) | |
|---|---|---|
| | J21 | J42 |
| Placebo | -2,0 | -1,3 |
| Principe actif 2,5% | 7,7 | 13,2 |
| Variation / Placebo (%) | 9,7 | 14,5 |

Le principe actif selon l'invention formulé à 2,5% en émulsion favorise la restructuration de la matrice dermique altérée avec l'âge chez des volontaires matures caucasiens. Dès 21 jours de traitement, en comparaison avant le traitement, le principe actif selon l'invention permet d'allonger les fibres de la matrice dermique, améliorant ainsi la qualité des fibres la matrice dermique de 14,5%. Par ailleurs, la densité dermique est également augmentée de 7,7%. Ces effets s'intensifient après 42 jours de traitement.

### Essai 4 - Evaluation in vivo de l'efficacité du principe actif selon l'invention sur sa capacité à agir comme perfecteur de teint

### Essai 4.1 - Evaluation de l'éclat du teint

L'éclat du teint a été étudié par scorage clinique chez les sujets caucasiens jeunes et matures ainsi que chez les sujets afro-américains, asiatiques et hispano-américains à l'aide d'un Glossymeter.

Les résultats sont présentés en Figure 4.

Dès 15 minutes après application unique, une amélioration significative de l'éclat de la peau est mesurée pour les panels caucasiens, asiatique et afro-américain. Avantageusement, cet effet se poursuit et s'intensifie après 21 jours de traitement avec une augmentation significative du paramètre rayonnement de l'éclat du teint observée :
- chez les jeunes caucasiens : +10,9%,
- chez les jeunes afro-américains : +30,7%,
- chez les sujets matures caucasiens et asiatiques : respectivement de +11,2% et +19,4%.

Ainsi, le principe actif selon l'invention sublime l'éclat du teint de façon immédiate et continue. Cet effet est multi-ethnique.

### Essai 4.2 - Evaluation de l'hyperpigmentation post-inflammatoire sur les panels jeunes hispano-américain et afro-américain.

Le teint peut être affecté par la présence de tâches telles que des marques d'hyperpigmentation. La diminution de ces désordres pigmentaires permet d'homogénéiser le teint.

Les marques d'hyperpigmentation post-inflammatoires ont été étudiées par analyse d'images issues du panel hispano-américains et par scorage clinique chez les sujets afro-américains.

Les résultats sont présentés dans le tableau 8 ci-après.

**[Tableau 8]**

| | Variation / J0 (%) | |
|---|---|---|
| | J7 | J21 |
| Hispano-américain | -17,5 | -21,7 |
| Afro-américain | -8,9 | -16,6 |

Dès 7 jours, le principe actif selon l'invention réduit significativement la visibilité des marques d'hyperpigmentation post-inflammatoires récentes : de 17,5% chez les sujets hispano-américains et de 8,9% chez les sujets afro-américains. Cet effet s'accentue encore après 21 jours de traitement avec une diminution des désordres pigmentaires chez les sujets hispano-américains de 21,7% et chez les sujets afro-américains de 16,6%.

Le principe actif selon l'invention démontre ainsi un effet anti-désordres pigmentaires rapide et contribue ainsi à homogénéiser le teint. Cet effet est multi-ethnique.

### Essai 4.3 - Etude des tâches de sénescence sur les panels matures caucasien et asiatique

Le vieillissement naturel de la peau est un phénomène qui entraine notamment l'apparition de tâches de sénescence. Afin de conserver un teint uniforme, il est possible de chercher à réduire la visibilité de ces tâches.

Les tâches de sénescence ont été étudiées par analyse d'images issues de C-Cube chez les sujets caucasiens et par mesure à l'aide d'un Mexamètre chez les sujets asiatiques.

Les résultats sont présentés dans le tableau 9 ci-après.

**[Tableau 9]**

| | Variation / J0 (%) | |
|---|---|---|
| | J21 | J42 |
| Caucasien | -5,7 | -7,5 |
| Asiatique | -18,2 | -26,6 |

Dès 21 jours de traitement avec le principe actif selon l'invention, l'apparence des taches de sénescence est significativement réduite chez les volontaires matures caucasiens et asiatiques. Cet effet s'intensifie après 42 jours de traitement avec une diminution de la visibilité des taches de 7,5% chez les sujets caucasiens et de 26,6% chez les volontaires asiatiques.

Ainsi, le principe actif selon l'invention réduit également l'apparence des tâches sur les peaux matures et permet ainsi de conserver un teint unifié. Cet effet est multi-ethnique.

### Essai 5 - Evaluation de l'effet hydra-lissant du principe actif selon l'invention

### Essai 5.1 - Evaluation du taux d'hydratation sur les panels jeunes caucasien, hispano-américain et afro-américain.

L'étude du taux d'hydratation des panels jeunes caucasien, hispano-américain et afro-américain a été effectuée à l'aide d'un Cornéomètre.

Les résultats sont présentés dans le tableau 10 ci-après.

**[Tableau 10]**

| | Variation / J0 (%) | |
|---|---|---|
| | J7 | J21 |
| Caucasien | 14,6 | 17,7 |
| Hispano-américain | 43,3 | 65,9 |
| Afro-américain | 13,0 | 7,1 |

Dès 7 jours d'utilisation, une augmentation significative de l'hydratation cutanée avec le principe actif selon l'invention est mesurée. Le principe actif selon l'invention présente ainsi un effet hydratant. Cet effet est multi-ethnique.

### Essai 5.2 - Evaluation du microrelief sur les panels jeunes caucasien, hispano-américain et afro-américain

L'étude de l'effet lissant a été effectuée par projection de franges chez les sujets caucasiens, par analyse d'image chez les sujets hispano-américains et par scorage clinique chez les sujets afro-américains.

Les résultats sont présentés dans le tableau 11 ci-après.

**[Tableau 11]**

| | Variation / J0 (%) | |
|---|---|---|
| | J7 | J21 |
| Caucasien | -6,1 | -3,9 |
| Hispano-américain | 0 | -13,2 |
| Afro-américain | -9,5 | -26,2 |

Dès 7 jours d'utilisation, le principe actif selon l'invention favorise significativement le lissage du microrelief et des ridules chez les sujets jeunes caucasiens et afro-américains. Cet effet s'accentue après 21 jours de traitement chez les panels hispano-américain et afro-américain, avec une diminution significative de 13,2% et de 26,2%.

Le principe actif selon l'invention présente ainsi un effet lissant participant à une amélioration de la qualité de la surface de la peau, cet effet est multi-ethnique.

### Essai 5.3 - Evaluation du grain de peau sur les panels jeunes caucasien et afro-américain

L'étude de l'aspect du grain de peau a été effectuée par scorage clinique chez les sujets caucasiens et afro-américains et par analyse d'images chez les sujets hispano-américains.

Les résultats sont présentés dans le tableau 12 ci-après.

**[Tableau 12]**

| | Variation / J0 (%) | |
|---|---|---|
| | J7 | J21 |
| Caucasien | -12,7 | -14,3 |
| Hispano-américain | -10,6 | -13,1 |
| Afro-américain | -15,7 | -21,7 |

Dès 7 jours, le principe actif selon l'invention diminue significativement la visibilité des pores de la peau des sujets caucasiens de 12,7% et des sujets afro-américains de 15,7 % et tend à affiner les pores des sujets hispano-américains. Cet effet s'accentue après 21 jours de traitement avec une diminution significative de la visibilité des pores de 14,3% chez les volontaires caucasiens, et de 21,7% chez les sujets afro-américains. Une diminution de 13,1% a également pu être appréciée chez les sujets hispano-américains.

Ainsi, en diminuant la visibilité des pores, le principe actif selon l'invention affine le grain de peau et permet d'améliorer les désagréments esthétiques liés aux problèmes des peaux jeunes de différentes populations.

### Essai 6 - Evaluation de l'action anti-âge du principe actif selon l'invention

### Essai 6.1 - Evaluation de l'effet anti-rides sur les panels matures caucasien et asiatique

L'évaluation de l'effet anti-rides a été effectuée par projection de franges chez les sujets matures caucasiens et asiatiques.

Les résultats sont présentés dans le tableau 13 ci-après.

**[Tableau 13]**

| | Variation / J0 (%) | | |
|---|---|---|---|
| | J7 | J21 | J42 |
| Caucasien | -7,9 | -10,8 | -8,5 |
| Asiatique | -11,7 | -11,3 | -17,8 |

Le principe actif selon l'invention formulé à 2,5% présente un effet anti-rides chez les volontaires matures dès 7 jours d'utilisation avec une diminution significative des rides de 7,9% chez les volontaires caucasiens, de 11,7% chez les volontaires asiatiques. Cet effet se poursuit après 21 et 42 jours pour les deux panels. Ainsi, le principe actif selon l'invention présente un effet anti-ride rapide et durable, quel que soit le type de peaux.

### Essai 6.2 - Etude de la fermeté sur panels matures caucasien et asiatique

L'évaluation de la fermeté de la peau a été effectuée à l'aide d'un Cutomètre chez les sujets matures caucasiens et asiatiques.

Les résultats sont présentés dans le tableau 14 ci-après.

**[Tableau 14]**

| | Variation / J0 (%) | |
|---|---|---|
| | J21 | J42 |
| Caucasien | 12,7 | 15,7 |
| Asiatique | 4,1 | 10,8 |

Dès 21 jours de traitement, le principe actif selon l'invention formulé à 2,5% améliore significativement la fermeté de la peau des volontaires caucasiens de 12,7%. Cet effet s'intensifie après 42 jours de traitement avec une augmentation significative de la fermeté de 15,7% chez les volontaires caucasiens ; de 10,8% chez les volontaires asiatiques. Ainsi, en améliorant la fermeté des peaux matures quel que soit le type de peaux, le principe actif selon l'invention offre un puissant pouvoir anti-âge.

### Exemple 2 : Principe actif hors invention

Le produit de l'exemple 2 est obtenu à partir d'un extrait de l'espèce *Ceratonia siliqua* connu pour sa richesse en galactomannanes. Le produit de l'exemple 2 est obtenu par le procédé suivant :
a. Solubilisation des graines de *Ceratonia siliqua* dans l'eau à raison d'au moins 50 g/l,
b. Extraction par hydrolyses enzymatiques,
c. Séparation des phases soluble et insoluble,
d. Traitement thermique,
e. Concentration de la phase soluble.

Le produit selon l'exemple 3 est constitué d'un extrait de graines de *Ceratonia siliqua* possédant notamment les caractéristiques suivantes :
- Teneur en Matières Sèches = 61,4 g/L, dont :
- Teneur en protéines (dosage selon la méthode Lowry) = 7,2 g/l soit 12%, en poids par rapport à la matière sèche,
- Teneur en sucres totaux = 52 g/L, soit 85% en poids par rapport à la matière sèche,
- 100% ont une masse moléculaire comprise entre 360 Da et 100 kDa.

Les résultats d'efficacité, à savoir la synthèse de collagène I sur des fibroblastes humains, sont présentés dans le tableau 15 ci-après.

**[Tableau 15]**

| | **Synthèse de collagène I (ng/mg protéines)** | **Efficacité (%)** |
|---|---|---|
| Témoin | 344 | |
| INVENTION - Exemple 1 à 0,5% | 1005 | **+192** |
| INVENTION - Exemple 1 à 1,0% | 1425 | **+314** |
| HORS INVENTION - Exemple 2 à 0,5% | 297 | - |
| HORS INVENTION - Exemple 2 à 1,0% | 317 | - |

Ce principe actif obtenu par un procédé similaire à l'invention, mais sur une matière première différente ne montre aucune efficacité sur la synthèse de collagène I. Ainsi, l'activité du principe actif selon l'invention est liée à la taille et à la structure des galactomannanes spécifiques issus de *Medicago sativa.*

### Exemple 3 : Composition selon l'invention

Un exemple de formulation comprenant le principe actif selon l'invention sous forme d'un sérum anti-âge est présenté dans le tableau 16 ci-après.

**[Tableau 16]**

| | **Ingrédients** | **%** |
|---|---|---|
| A1 | Eau Purifiée | qsp 100 |
| | Conservateur | qs |
| | Erythritol | 3,00 |
| A2 | Sclerotium Gum & Xanthan Gum | 0,30 |
| B | Steareth-2 | 1,84 |
| | Steareth-21 | 1,16 |
| | Caprylyl Methicone | 5,00 |
| | Dimethicone | 10,00 |
| C | Dimethicone & Dimethicone/Vinyl Dimethicone Crosspolymer | 6,00 |
| D | Polyacrylate-13 & Polyisobutene & Polysorbate 20 | 0,50 |
| E | **Principe actif Invention** | **2,50** |

La composition de l'exemple 3 peut notamment être obtenue par le procédé suivant :
- Ajouter A2 dans A1 sous agitation et chauffer à 70°C.
- Placer B sous agitation et chauffer à 70°C.
- Emulsionner B dans A sous agitation cisaillante pendant 10 minutes.
- A 60°C, ajouter C sous agitation cisaillante. Agiter pendant 5 minutes.
- A température ambiante, sous agitation douce, ajouter D puis E.

La composition se présente alors sous forme d'une émulsion fluide, brillante de couleur blanc cassé.

### Exemple 4 : Composition selon l'invention

Un exemple de formulation comprenant le principe actif selon l'invention sous forme d'une crème onctueuse raffermissante est présenté dans le tableau 17 ci-après.

**[Tableau 17]**

| | **Ingrédients** | **%** |
|---|---|---|
| A1 | Eau Purifiée | qsp 100 |
| | Conservateur | qs |
| | Butylène Glycol | 4,00 |
| A2 | Glycerin | 2,00 |
| | Sclerotium Gum & Xanthan Gum | 0,30 |
| B | Cetearyl Alcohol & Cetearyl Glucoside | 2,00 |
| | Coco-Glucoside & Coconut Alcohol | 2,00 |
| | C10-18 Triglycerides | 1,00 |
| | Oleyl Erucate | 5,00 |
| | Isocetyl Stéarate | 4,00 |
| | Dicaprylyl Carbonate | 6,00 |
| | Propanediol Dicaprylate | 4,00 |
| | Dimethicone | 2,00 |
| | Acrylates/C10-30 Alkyl Acrylate Crosspolymer | 0,30 |
| C | **Principe actif Invention** | **2,50** |
| D | Solution Soude 28% | qsp pH |

La composition de l'exemple 4 peut notamment être obtenue par le procédé suivant :
- Ajouter A2 dans A1 sous agitation et chauffer à 80°C
- Placer B sous agitation et chauffer à 80°C.
- Emulsionner B dans A sous agitation cisaillante pendant 10 minutes.
- A température ambiante, sous agitation douce, ajouter C.
- Ajuster le pH à 5,0 - 5,5 avec D.

La composition se présente alors sous forme d'une émulsion très épaisse de couleur blanc cassé.

### Exemple 5 : Composition selon l'invention

Un exemple de formulation comprenant le principe actif selon l'invention sous forme d'un gel crème lissant est présenté dans le tableau 18 ci-après.

**[Tableau 18]**

| | **Ingrédients** | **%** |
|---|---|---|
| A | Eau Purifiée | qsp 100 |
| | Butylène Glycol | 3,00 |
| | Glycerin | 2,00 |
| | Propylene Glycol | 2,00 |
| B | Acrylates/C10-30 Alkyl Acrylate Crosspolymer | 0,40 |
| C | Caprylic/Capric/Succinic Triglyceride | 4,00 |
| | Dicaprylyl Carbonate | 4,00 |
| | Octyldodecanol | 4,00 |
| | Ricinus Communis (Castor) Seed Oil | 2,00 |
| | Tocopherol | 0,01 |
| D | Solution Soude 28% | qsp pH |
| E | Conservateur | qs |
| F | **Principe actif Invention** | **2,50** |
| G | Silica | 2,00 |

La composition de l'exemple 5 peut notamment être obtenue par le procédé suivant :
- Disperser B dans A sous agitation modérée.
- Ajouter sous agitation vive C dans AB.
- Ajuster le pH avec D à 5,0 - 5,5.
- Maintenir l'agitation pendant 10 minutes.
- Sous agitation modérée, ajouter E, F et G.

La composition se présente alors sous forme d'un gel crème brillant et de couleur blanc cassé.

### Exemple 6 : Composition selon l'invention

Un exemple de formulation comprenant le principe actif selon l'invention sous forme d'un fond de teint anti-rides est présenté dans le tableau 19 ci-après.

**[Tableau 19]**

| | **Ingrédients** | **%** |
|---|---|---|
| A | Tri(Polyglyceryl-3/Lauryl) Hydrogenated Trilinoleate | 3,00 |
| | Cera Alba (Beeswax) | 0,40 |
| | Magnésium Stéarate | 0,25 |
| | C12-15 Alkyl Benzoate & Stearalkonium Hectorite & Propylene Carbonate | 7,00 |
| | Diisopropyl Sebacate | 13,00 |
| B | Crambe Abyssinica Seed Oil | 7,00 |
| | CI 77499 (Iron Oxides) & Isostearyl | 0,15 |
| | Sebacate & Disodium Stearoyl Glutamate & Aluminum Hydroxide | |
| | CI 77491 (Iron Oxides) & Isostearyl Sebacate & Disodium Stearoyl Glutamate & Aluminum Hydroxide | 0,30 |
| | CI 77891 (Titanium Dioxide) & Isostearyl Sebacate & Disodium Stearoyl Glutamate & Aluminum Hydroxide | 7,00 |
| | CI 77492 (Iron Oxides) & Isostearyl Sebacate & Disodium Stearoyl Glutamate& Aluminum Hydroxide | 0,70 |
| C | Eau Purifiée | qsp 100 |
| | Conservateur | qs |
| | Glycérine | 4,00 |
| | Magnésium Sulfate | 1,00 |
| D | Kaolin | 2,50 |
| E | **Principe actif Invention** | **2,50** |

La composition de l'exemple 6 peut notamment être obtenue par le procédé suivant :
- Chauffer A à 80°C et homogénéiser sous agitation vive.
- Broyer B à la tricylindre et transférer dans A. Agiter jusqu'à homogénéité.
- Placer C sous agitation et chauffer à 80°C.
- Ajouter très lentement C dans AB sous agitation vive.
- Homogénéiser pendant 10 minutes sous agitation cisaillante.
- A 30°C, ajouter D puis E et homogénéiser pendant 1 minutes sous agitation cisaillante.

La composition se présente alors sous forme d'une émulsion épaisse et brillante de couleur beige clair.

## Revendications

1. Utilisation cosmétique d'un principe actif comprenant des galactomannanes issus d'un hydrolysat obtenu à partir des graines de *Medicago sativa,* pour son effet perfecteur de teint sur les peaux saines.

2. Utilisation cosmétique d'un principe actif selon la revendication précédente, **caractérisée en ce que** les peaux saines sont des peaux saines caucasiennes et asiatiques et afro-américaines et hispano-américains.

3. Utilisation cosmétique d'un principe actif selon la revendication précédente **caractérisée en ce qu'**il présente des effets perfecteur de teint et hydratant et anti-âge.

4. Utilisation cosmétique d'un principe actif selon l'une des revendications précédentes, **caractérisé en ce que** la fraction sucre de l'hydrolysat comprend au moins 60% de galactomannanes en poids du poids total des sucres de l'extrait.

5. Principe actif cosmétique comprenant au moins un extrait obtenu à partir des graines de *Medicago sativa,* **caractérisé en ce que** l'extrait de *Medicago sativa* comprend, en poids du poids total de l'extrait, au moins 35% de sucres et au plus 25% de protéines.

6. Principe actif cosmétique selon la revendication précédente, **caractérisé en ce que** la fraction sucre de l'extrait comprend au moins 60% de galactomannanes en poids du poids total des sucres de l'extrait.

7. Principe actif cosmétique selon l'une des revendications 5 ou 6, **caractérisé en ce que** la fraction sucre de l'extrait comprend au moins 60% de galactomannanes de masses moléculaires comprises entre 360 Da (DP2) et 1260 kDa (DP7000), en poids du poids total des sucres de l'extrait.

8. Principe actif cosmétique selon l'une des revendications 5 à 7, **caractérisé en ce que** la fraction sucre de l'extrait comprend au moins 30% de galactomannanes de masses moléculaires comprises entre 3,6 kDa (DP20) et 1260 kDa (DP7000), en poids du poids total des sucres de l'extrait.

9. Principe actif cosmétique selon l'une des revendications 5 à 8, **caractérisé en ce que** l'extrait de *Medicago sativa* est un hydrolysat.

10. Principe actif pour son utilisation selon la revendication précédente, **caractérisé en ce que** l'hydrolysat de *Medicago sativa* est un hydrolysat enzymatique.

11. Principe actif cosmétique selon l'une des revendications 5 à 10, **caractérisé en ce que** l'extrait est susceptible d'être obtenu par le procédé comprenant les étapes suivantes :
a. Solubilisation des graines de *Medicago sativa* dans l'eau à raison d'au moins 50 g/I,
b. Extraction par hydrolyse, préférentiellement hydrolyses enzymatiques,
c. Séparation des phases soluble et insoluble,
d. Traitement thermique,
e. Concentration de la phase soluble,
f. Purification par chromatographie ionique,
g. Éventuellement, filtration et filtration stérilisante.

12. Composition cosmétique comprenant au moins 0,1% d'un principe actif cosmétique selon l'une des revendications 5 à 11, en poids du poids total de la composition.
